# EUROPEAN PATENT SPECIFICATION

(11) **EP 1 253 206 B1**
(45) Date of publication and mention of the grant of the patent: **11.07.2007**
(21) Application number: 02009618.6
(22) Date of filing: 26.04.2002
(51) Int. Cl.: C12Q 1/68, C12Q 1/70

(54) **METHOD OF AMPLIFYING OR DETECTING HIV-1 RNA**
VERFAHREN ZUR AMPLIFIZIERUNG ODER ZUM NACHWEIS VON HIV-1-RNA
PROCEDE D'AMPLIFICATION OU DE DETECTION DE L'ARN DU VIH-1

(30) Priority: 26.04.2001 JP 2001129210
(43) Date of publication of application: 30.10.2002
(73) Proprietor: TOSOH CORPORATION, Shinnanyo-shi, Yamaguchi-ken, 746-8501 (JP)
(72) Inventor: Ishizuka, Tetsuya, Sagamihara-shi, Kanagawa-ken (JP); Yasukawa, Kiyoshi, Kawasaki-shi, Kanagawa-ken (JP); Ishiguro, Takahiko, Yokohama-shi, Kanagawa-ken (JP)
(74) Representative: Vossius & Partner

(56) References cited:
- EP-A- 0 806 484
- EP-A- 0 969 101
- WO-A-93/08836
- WO-A-95/11701
- US-A- 5 374 524
- US-A- 5 707 864
- KIEVITS T ET AL: "NASBA TM ISOTHERMAL ENZYMATIC IN VITRO NUCLEIC ACID AMPLIFICATION OPTIMIZED FOR THE DIAGNOSIS OF HIV-1 INFECTION" JOURNAL OF VIROLOGICAL METHODS, AMSTERDAM, NL, vol. 35, no. 3, 1 December 1991 (1991-12-01), pages 273-286, XP000576430 ISSN: 0166-0934
- BRUISTEN S ET AL: "DETECTION OF HIV-1 DISTRIBUTION IN DIFFERENT BLOOD FRACTIONS BY TWONUCLEIC ACID AMPLIFICATION ASSAYS" AIDS RESEARCH AND HUMAN RETROVIRUSES, NEW YORK, NY, US, vol. 9, no. 3, 1993, pages 259-266, XP002932546 ISSN: 0889-2229
- ROMANO J W ET AL: "NASBA A NOVEL, ISOTHERMAL DETECTION TECHNOLOGY FOR QUALITATIVE AND QUANTITATIVE HIV-1 RNA MEASUREMENTS" CLINICS IN LABORATORY MEDICINE, W.B. SAUNDERS CO., LONDON, GB, vol. 16, no. 1, 1 March 1996 (1996-03-01), pages 89-103, XP000600141 ISSN: 0272-2712

### SEQUENCE LISTING

<110> Tosoh Corporation
<120>H I V - 1 METHOD OF AMPLIFYING OR DETECTING HIV-1 RNA
<130> 211-0459
<160> 35
<210> 1
   <211> 20
   <212> DNA
   <213> Artificial Sequence
<220>
   <223> First primer
<400> 1
   actgtattat ataatgatct 20
<210> 2
   <211> 20
   <212> DNA
   <213> Artificial Sequence
<220>
   <223> First primer
<400> 2
   attatataat gatctaagtt 20
<210> 3
   <211> 20
   <212> DNA
   <213> Artificial Sequence
<220>
   <223> First primer
<400> 3
   ataatgatct aagttcttct 20
<210> 4
   <211> 20
   <212> DNA
   <213> Artificial Sequence
-<220>
   <223> First primer
<400> 4
   gagggttgct actgtattat 20
<210> 5
   <211> 20
   <212> DNA
   <213> Artificial Sequence
<220>
   <223> First primer
<400> 5
   caatagaggg ttgctactgt 20
<210> 6
   <211> 20
   <212> DNA
   <213> Artificial Sequence
<220>
   <223> First primer
<400> 6
   gcacacaata gagggttgct 20
<210> 7
   <211> 20
   <212> DNA
   <213> Artificial Sequence
<220>
   <223> First primer
<400> 7
   ttgctactgt attatataat 20
<210> 8
   <211> 25
   <212> DNA
   <213> Artificial Sequence
<220>
   <223> Second primer
<400> 8
   ggaaagaaaa aatataaatt aaaac 25
<210> 9
   <211> 25
   <212> DNA
   <213> Artificial Sequence
<220>
   <223> Second primer
<400> 9
   gaaaaaatat aaattaaaac atata 25
<210> 10
   <211> 25
   <212> DNA
   <213> Artificial Sequence
<220>
   <223> Second primer
<400> 10
   aaaaatataa attaaaacat atagt 25
<210> 11
   <211> 25
   <212> DNA
   <213> Artificial Sequence
<220>
   <223> Second primer
<400> 11
   aaaatataaa ttaaaacata tagta 25
<210> 12
   <211> 25
   <212> DNA
   <213> Artificial Sequence
<220>
   <223> Second primer
<400> 12
   aaatataaat taaaacatat agtat 25
<210> 13
   <211> 25
   <212> DNA
   <213> Artificial Sequence
<220>
   <223> Second primer
<400> 13
   aatataaatt aaaacatata gtatg 25
<210> 14
   <211> 25
   <212> DNA
   <213> Artificial Sequence
<220>
   <223> Second primer
<400> 14
   atataaatta aaacatatag tatgg 25
<210> 15
   <211> 25
   <212> DNA
   <213> Artificial Sequence
<220>
   <223> Second primer
<400> 15
   tataaattaa aacatatagt atggg 25
<210> 16
   <211> 25
   <212> DNA
   <213> Artificial Sequence
<220>
   <223> Second primer
<400> 16
   ataaattaaa acatatagta tgggc 25
<210> 17
   <211> 25
   <212> DNA
   <213> Artificial Sequence
<220>
   <223> Second primer
<400> 17
   aaattaaaac atatagtatg ggcaa 25
<210> 18
   <211> 25
   <212> DNA
   <213> Artificial Sequence
<220>
   <223> Second primer
<400> 18
   aaaacatata gtatgggcaa gcagg 25
<210> 19
   <211> 25
   <212> DNA
   <213> Artificial Sequence
<220>
   <223> Second primer
<400> 19
   atatagtatg ggcaagcagg gagct 25
<210> 20
   <211> 25
   <212> DNA
   <213> Artificial Sequence
<220>
   <223> Second primer
<400> 20
   gtatgggcaa gcagggagct agaac 25
<210> 21
   <211> 20
   <212> DNA
   <213> Artificial Sequence
<220>
   <223> Third oligonucleotide
<400> 21
   tttccccctg gccttaaccg 20
<210> 22
   <211> 20
   <212> DNA
   <213> Artificial Sequence
<220>
   <223> Third oligonucleotide
<400> 22
   ttttctttcc ccctggcctt 20
<210> 23
   <211> 20
   <212> DNA
   <213> Artificial Sequence
<220>
   <223> Third oligonucleotide
<400> 23
   ttttttcttt ccccctggcc 20
<210> 24
   <211> 20
   <212> DNA
   <213> Artificial Sequence
<220>
   <223> Third oligonucleotide
<400> 24
   attttttctt tccccctggc 20
<210> 25
   <211> 20
   <212> DNA
   <213> Artificial Sequence
<220>
   <223> Third oligonucleotide
<400> 25
   tattttttct ttccccctgg 20
<210> 26
   <211> 20
   <212> DNA
   <213> Artificial Sequence
<220>
   <223> Third oligonucleotide
<400> 26
   atattttttc tttccccctg 20
<210> 27
   <211> 20
   <212> DNA
   <213> Artificial Sequence
<220>
   <223> Third oligonucleotide
<400> 27
   tatatttttt ctttccccct 20
<210> 28
   <211> 20
   <212> DNA
   <213> Artificial Sequence
<220>
   <223> Third oligonucleotide
<400> 28
   ttatattttt tctttccccc 20
<210> 29
   <211> 20
   <212> DNA
   <213> Artificial Sequence
<220>
   <223> Third oligonucleotide
<400> 29
   tttatatttt ttctttcccc 20
<210> 30
   <211> 20
   <212> DNA
   <213> Artificial Sequence
<220>
   <223> Third oligonucleotide
<400> 30
   aatttatatt ttttctttcc 20
<210> 31
   <211> 20
   <212> DNA
   <213> Artificial Sequence
<220>
   <223> Third oligonucleotide
<400> 31
   gttttaattt atattttttc 20
<210> 32
   <211> 20
   <212> DNA
   <213> Artificial Sequence
<220>
   <223> Third oligonucleotide
<400> 32
   tatatgtttt aatttatatt 20
<210> 33
   <211> 20
   <212> DNA
   <213> Artificial Sequence
<220>
   <223> Third oligonucleotide
<400> 33
   catactatat gttttaattt 20
<210> 34
   <211> 19
   <212> DNA
   <213> Artificial Sequence
<220>
   <223> Oligonucleotide probe
<400> 34
   tctgaaggga tggttgtag 19
<210> 35
   <211> 28
   <212> DNA
   <213> Artificial Sequence
<220>
   <223> T 7 polymerase promoter sequence
<400> 35
   aattctaata cgactcacta tagggaga 28

## Description

The present invention relates to methods for the amplification or detection of HIV-1 RNA in clinical tests and diagnoses.

Human immunodeficiency virus (HIV) is the pathogen of acquired immunodeficiency syndrome (AIDS). Two subtypes of HIV are known: HIV-1, which is spread worldwide, and HIV-2, which is epidemic mainly on the African West coast. The similarity between HIV-2 and simian immunodeficiency virus (SIV) in base sequence implies that HIV-2 may be zoonotic. However, clinical conditions of HIV-2 infection are less serious than those of HIV-1 infection. HIV-1 infection induces the Production of antibodies against structural proteins and regulatory proteins of HIV-1. HIV-1 attacks the T cells classified as CD4+ lymphocytes as the main target immunocytes and hence abnormalizes the immune system in various ways. In the advanced stages of HIV-1 infection, B cells are stimulated to set off hypergammaglobulinemia, and autoantibodies and immunocomplexes appear with a marked reduction of lymphocytes and blood platelets. Complications such as tuberculosis, Pneumocystis carinii pneumonia and other opportunistic infections at high levels of immunodeficiency induced by HIV-1 infection are diagnostic of onset of AIDS.

For diagnosis of HIV-1 infection, EIA (enzyme immunoassay) based on colorimetric detection of the reaction of an antibody against a viral antigen is available, coupled with Western blot confirmation of suspected positive serum samples by the presence of antibodies in the serum samples which react to a specific virus antigen in a blot of electrophoretically separated various virus particle antigens. However, assay methods which detect antibodies like these are not available for diagnoses of early stage infection before the production of antibodies.

As discussed above, conventional assay methods often fail to detect HIV-1 in the early stage of infection, require complicated operations and long time and can hardly detect trace amounts of HIV-1 in a sample.

Therefore, the development of a speedy and sensitive detection method is demanded. Especially, quantification of HIV-1 RNA is crucial for obtaining information about the pathological progress and the effectiveness of anti-HIV drugs. Further, the development of automatic analyzers is demanded to facilitate clinical tests.

For high sensitive detection, it is preferred to amplify a specific sequence in a gene to be detected or identified or an RNA derived from such a gene.

As a method of amplifying a specific sequence in RNAs like the HIV-1 genomic RNA, the reverse transcription-polymerase chain reaction (RT-PCR) is known. In this method, the reverse transcription step for synthesis of the cDNA of the target RNA is followed by repeated cycles of heat denaturation, primer-annealing and elongation reaction in the presence of a pair of primers, one of which is complementary to either end of the specific sequence and the other is homologous to the other end of the specific sequence (the antisense primer may be the same as the primer used in the reverse transcription step), and a thermostable DNA polymerase to give DNA as the amplification product of the specific sequence.

However, the necessity to conduct the operations in two steps (the reverse transcription step and the PCR step) and to repeat the cumbersome operations such as rapid heating and cooling hinders automation of the RT-PCR.

NASBA or 3SR is known as a technique for amplifying a specific RNA sequence by the cooperative action of a reverse transcriptase and an RNA polymerase. This technique involves a chain reaction comprising the synthesis of a double-stranded DNA from the target RNA. By using a primer containing promoter sequences, the target RNA is reverse-transcribed by the use of a reverse transcriptase followed by hydrolysis of the RNA portion by the use of ribonuclease H.

By the help of a second primer and the DNA dependent DNA polymerase activity of the reverse transcriptase, a DNA strand complementary to the first DNA strand is produced. Using the resulting double-stranded DNA containing the promoter sequence as a template, an RNA polymerase produces an RNA having the specific sequence. NASBA or 3SR allows relatively isothermal amplification of ribonucleic acids and is considered suitable for automation.

However, since the reactions involved in this amplification technique are carried out at relatively low temperatures (for example, at 41°C), formation of intramolecular secondary structure of the target RNA may occur, thus preventing binding of the primers and reducing the reaction efficiency. Therefore, it is necessary to increase the binding efficiency of the primers before the amplification reaction. This is achieved by resolving the intramolecular secondary structure of the target RNA, for example, by heat denaturation of the target RNA.

The object of the present invention is to provide a simple, speedy and sensitive method for amplifying or detecting HIV-RNA.

The present invention has been accomplished to attain the above-mentioned object by the provision of an oligonucleotide that can bind to a region of the genomic RNA of HIV-1 which is free of RNA secondary structure.

Therefore, the oligonucleotide can be used as a primer at relatively low and constant temperatures (at 35°C to 50°C, preferably at 41°C) for amplification of a nucleic acid.

Specifically, the invention defined in the claims of the present application provides a method of amplifying an RNA of HIV-1, anticipated in a sample as a template which comprises the steps of
a) synthesizing a cDNA by the action of an RNA-dependent DNA polymerase, by using a first primer containing a sequence complementary to a specific sequence of said RNA,
b) denuding the cDNA to a single-stranded DNA through degradation of the RNA in the resulting RNA-DNA double strand by ribonuclease H activity,
c) forming a double-stranded DNA having a promoter sequence which can be transcribed into an RNA consisting of the specific sequence or a sequence complementary to the specific sequence by using the single-stranded DNA as template and by the action of a DNA-dependent DNA polymerase,
d) transcribing a strand.of the double-stranded DNA into an RNA transcript, by.the use of an RNA polymerase and
e) synthesizing a cDNA of the RNA transcript of step (c) by the RNA-dependent DNA polymerase, in the presence of the RNA polymerase,
wherein either the first or the second primer additionally contains a promoter sequence for the RNA polymerase at the 5 end and wherein the first primer is an oligonucleotide comprising the sequence of SEQ ID NO: 2,
and the second primer is an oligonucleotide comprising the sequence of SEQ ID NO: 13.

The method of the invention preferably further comprises adding a third oligonulcleotide which is complementary to a region of the RNA of HIV-1 which flanks the 5' end of the specific sequence with an overlap of from 1 to 10 bases with the specific sequence to form a template used in the initial stage of the amplification by cutting the RNA derived from HIV-1 at the 5' end of the specific sequence by the action of the ribonuclease H activity, wherein the first primer is an oligonucleotide of SEQ ID NO: 2, and the second primer is an oligonucleotide of SEQ ID NO: 13, and the third oligonucleotide is an oligonucleotide of SEQ ID NO: 26.

In addition to the above, it is also disclosed herein that the first primer is an oligonucleotide of any one of SEQ ID NOs:1 to 7 and
(1) the second primer is an oligonucleotide of SEQ ID NO: 8 and the third oligonucleotide is an oligonucleotide of any one of SEQ ID NOs: 21 and 2,
(2) the second primer is an oligonucleotide of SEQ ID NO:9, and the third oligonucleotide is an oligonucleotide of any one of SEQ ID NOS:22 to 26,
(3) the second primer is an oligonucleotide of SEQ ID NO:10, and the third oligonucleotide is an oligonucleotide of any one of SEQ ID NOS: 22 to 28,
(4) the second primer is an oligonucleotide of SEQ ID NO:11, and the third oligonucleotide is an oligonucleotide of any one of SEQ ID NOS:22 to 29,
(5) the second primer is an oligonucleotide of SEQ ID NO:12, and the third oligonucleotide is an oligonucleotide of any one of SEQ ID NOS:-22 to 29,
(6) the second primer is an oligonucleotide of SEQ ID NO:13, and the third oligonucleotide is an oligonucleotide of any one of SEQ ID NOS: 23 to 30
(7) the second primer is an oligonucleotide of SEQ ID NO:14, and the third oligonucleotide is an oligonucleotide of any one of SEQ ID NOS:23 to 30,
(8) the second primer is an oligonucleotide of SEQ ID NO:15, and the third oligonucleotide is an oligonucleotide of any one of SEQ ID NOS:24 to 30,
(9) the second primer is an oligonucleotide of SEQ ID NO:16, and the third oligonucleotide is an oligonucleotide of any one of SEQ ID NOS:25 to 30,
(10) the second primer is an oligonucleotide of SEQ ID NO:17, and the third oligonucleotide is an oligonucleotide of any one of SEQ ID NOS:27 to 31,
(11) the second primer is an oligonucleotide of SEQ ID NO:18, and the third oligonucleotide is an oligonucleotide of any one of SEQ ID NOS:31 and 32,
(12) the second primer is an oligonucleotide of SEQ ID NO:19, and the third oligonucleotide is an oligonucleotide of any one of SEQ ID NOS:32 and 33, or
(13) the second primer is an oligonucleotide of SEQ ID NO:20, and the third oligonucleotide is an oligonucleotide of SEQ ID NO:33.

In addition, the invention provides a method of detecting HIV-1, which comprises conducting the aforementioned method of amplifying HIV-1 RNA in the presence of an oligonucleotide probe having a sequence different from those of the first primer and the second primer which can specifically bind to the RNA transcript resulting from the amplification and which is labeled with a fluorescent intercalative dye, and measuring the change in the fluorescence of the reaction solution. wherein the oligonucleotide probe is designed to hybridize with at least part of the RNA transcript and to alter its fluorescence upon hybridization and comprises a sequence consisting of or being complementary to at least 10 consecutive nucleotides in SEQ ID NO: 34.

Furthermore, the invention provides the use of an oligonucleotide of SEQ ID NOs: 2 and 13 for the amplification of HIV-1 RNA. In addition, herein disclosed is the use of an oligonucleotide of any one of SEQ ID NOs: 1 to 35 for the amplification or detection of HIV-1 RNA.

Also provided by the invention is the use of an oligonucleotide of SEQ ID NOs: 2, 13 and 34 for the detection of HIV-1 in a sample.

In yet another embodiment, the invention provides a kit comprising at least one oligonucleotide of any one of SEQ ID NOs: 2, 13, and 34 in one or more container. Preferably, said kit is for use in one of the above-mentioned methods and/or uses and consequently may additionally comprise further suitable ingredients such as the required enzymes, buffers, and the like as well as a corresponding manual describing said method or use to the user of the kit.

The figures show:

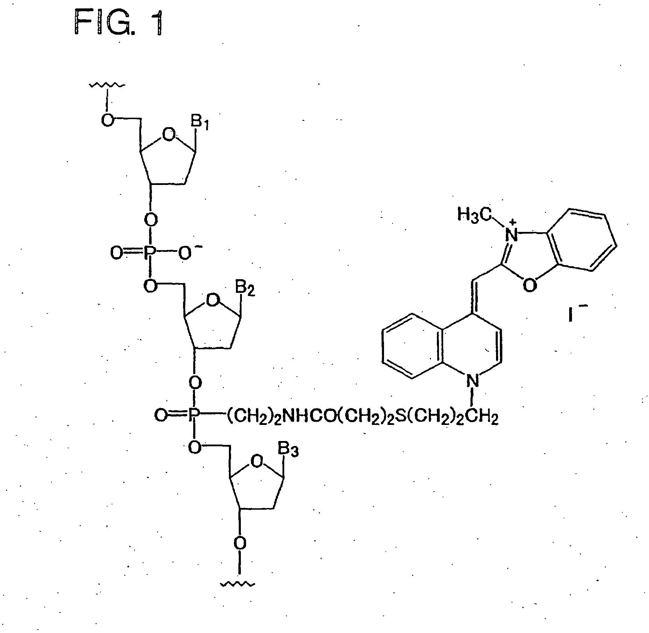

Fig. 1 is the chemical formula of the fluorescent intercalative dye moiety of the fluorescent intercalative dye-labeled oligonucleotide used in Example 2. B₁ to B₃ are nucleic acid bases.
Fig. 2 is a graph correlating the reaction time and the fluorescence enhancement accompanying RNA synthesis at initial RNA amounts of 10⁵ copies/30 µl to 10 copies/30 µl in Example 1. "Nega" indicates a sample prepared by using a diluent instead of the RNA sample.
Fig. 3 is a calibration curve obtained by plotting the detection time, which is defined as the time at the ratio of fluorescent intensity of 1.2, as ordinate and the initial RNA concentration as abscissa.
Fig. 4 is a graph correlating the reaction time and the fluorescence enhancement in the detection of HIV-RNA in the nucleic acid extracted from HIV-positive serum in Example 2.

The present invention provides a nucleic acid amplification method for amplification of HIV-RNA in a sample, and a method of detecting the RNA transcript formed by the nucleic acid amplification method. The amplification method of the present invention may cover any amplification methods such as PCR, NASBA or 3SR. However, isothermal nucleic acid amplification by the cooperative action of a reverse transcriptase and an RNA polymerase as, e.g. in NASBA or 3SR is preferred for amplifying a specific RNA sequence in HIV-1.

For example, NASBA amplification of an HIV-1 RNA present in a sample comprises synthesizing a cDNA by the action of an RNA-dependent DNA polymerase by using a specific sequence in said HTV-1 RNA as the template, subsequently, the cDNA is denuded by the action of ribonuclease H to a single-stranded DNA, through degradation of the RNA in the RNA-DNA double strand of the cDNA. NASBA amplification further comprises forming a double-stranded DNA comprising a promoter sequence which can be transcribed into an RNA consisting of the specific nucleotide sequence of HIV-1 or a sequence complementary to the specific nucleotide sequence. The formation of the double-stranded DNA is accomplished by using the single-stranded DNA as the template and by the action of a DNA-dependent DNA polymerase. Transcribing the double-stranded DNA into an RNA transcript generates further templates for subsequent cDNA syntheses by the RNA-dependent DNA polymerase. The present invention is characterized by the use of an oligonucleotide primer comprising a sequence of any one of SEQ ID NOs : 1 to 7 as a first primer, which can bind to a specific site of the HIV-1 RNA and an oligonucleotide comprising a sequence of any one of SEQ ID NOs: 8 to 20 as a second primer, which is homologous to the part of the HIV-1 RNA to be amplified. Additionally, one of said primers has a promoter sequence for the RNA polymerase at the 5' end.

One embodiment of the present invention is the above-mentioned amplification method, wherein the first primer is an oligonucleotide of SEQ ID NO: 2, and the second primer is an oligonucleotide of SEQ ID NO: 13, wherein either the first primer or the second primer additionally has a promoter sequence for the RNA polymerase at the 5' end. The RNA-dependent DNA polymerase, the DNA-dependent DNA polymerase and the ribonuclease H are not particularly limited, but AMV reverse transcriptase is preferable because it has the activities of all of them. As the RNA polymerase, T7 phage RNA polymerase or SP6 phage RNA polymerase is preferred, though there is no particular restriction.

In the above-mentioned amplification method, even if the specific sequence is not present at the 5' end, HIV-1 RNA can be amplified. Before the HIV-1 RNA is used as a template in the initial stage of the nucleic acid amplification, it's 5' end is cleaved off. This is achieved by adding an oligonucleotide complementary to a region of HIV-1 RNA which flanks the 5' end of the specific sequence and overlaps by 1 to 10 bases with the specific sequence. Cleavage is executed by ribonuclease H activity.

An oligonucleotide comprising the sequence of SEQ ID NO: 26 may be used as a mediator of cleavage. The cleavage-mediating oligonucleotide is preferred to have a chemically modified hydroxyl group (for example, an aminated hydroxyl group) at the 3' end which cannot be elongated from the 3' end.

Other cleavage-mediating oligonucleotides are those shown in SEO ID NOs: 21 to 25 and 27 to 33.

The third oligonucleotide is complementary to a region which flanks the 5' end of the specific HIV-1 sequence and overlaps with this region by 1 to 10 bases. This oligonucleotide is added to mediate cleavage of the HIV-1 RNA at the 5' end, by the action of a ribonuclease H activity. When this oligonucleotide is added, preferably before the RNA is used as the template, in the initial stage of the nucleic acid amplification, it is preferred that the first primer is an oligonucleotide of SEQ ID NO: 2, and the second primer is an oligonucleotide of SEQ ID NO: 13 and the third oligonucleotide is an oligonucleotide of SEQ ID NO: 26. In addition to the above, also disclosed herein is that the first primer is an oligonucleotide of any one of SEQ ID NOs: 1 to 7,
(1) the second primer is an oligonucleotide of SEQ ID NO:8, and the third oligonucleotide is an oligonucleotide of any one of SEQ ID NOS:21 and 22,
(2) the second primer is an oligonucleotide of SEQ ID NO:9, and the third oligonucleotide is an oligonucleotide of any one of SEQ ID NOS:22 to 26,
(3) the second primer is an oligonucleotide of SEQ ID NO:10, and the third oligonucleotide is an oligonucleotide of any one of SEQ ID NOS:22 to 28,
(4) the second primer is an oligonucleotide of SEQ ID NO:11, and the third oligonucleotide is an oligonucleotide of any one of SEQ ID NOS:22 to 29,
(5) the second primer is an oligonucleotide of SEQ ID NO:12, and the third oligonucleotide is an oligonucleotide of any one of SEQ ID NOS:22 to 29,
(6) the second primer is an oligonucleotide of SEQ ID NO:13, and the third oligonucleotide is an oligonucleotide of any one of SEQ ID NOS:23 to 30,
(7) the second primer is an oligonucleotide of SEQ ID NO:14, and the third oligonucleotide is an oligonucleotide of any one of SEQ ID NOS:23 to 30,
(8) the second primer is an oligonucleotide of SEQ ID NO:15, and the third oligonucleotide is an oligonucleotide of any one of SEQ ID NOS:24 to 30,
(9) the second primer is an oligonucleotide of SEQ ID NO:16, and the third oligonucleotide is an oligonucleotide of any one of SEQ ID NOS:25 to 30,
(10) the second primer is an oligonucleotide or SEQ ID NO:17, and the third oligonucleotide is an oligonucleotide of any one of SEQ ID NOS:27 to 31,
(11) the second primer is an oligonucleotide of SEQ ID NO:18, and the third oligonucleotide is an oligonucleotide of any one of SEQ ID NOS:31 and 32,
(12) the second primer is an oligonucleotide of SEQ ID NO:19, and the third oligonucleotide is an oligonucleotide of any one of SEQ ID NOS:32 and 33, or
(13) the second primer is an oligonucleotide of SEQ ID NO:20, and the third oligonucleotide is an oligonucleotide of SEQ ID NO:33.

In this case, the third oligonucleotide (the cleavage-mediating oligonucleotide) is preferred to have a chemically modified hydroxyl group (for example, an aminated hydroxyl group) at the 3' end which cannot be elongated form the 3' end, too.

Detection of the amplification product obtained in the nucleic acid amplification step is preferably carried out by measuring the change in the fluorescence of the reaction solution during the amplification step. Preferably, this is achieved by performing the amplification reaction in the presence of an oligonucleotide probe labeled with a fluorescent intercalative dye. However, the probe can also be detected by other methods for detection of nucleic acids. The oligonucleotide probe may be, for example, an oligonucleotide giving a fluorescent intercalative dye linked to a phosphorus atom via a linker. Such a preferable probe alters its fluorescence upon formation of a double strand with the complementary target nucleic acid by intercalation of the intercalating moiety into the double strand (Ishiguro, T. et al., (1996) Nucleic Acids Res. 24 (24) 4992-4997).

The sequence of the probe is not particularly limited as long as it contains a sequence complementary to at least part of the RNA transcript. For example, when the combination of.a first primer comprising a sequence of SEQ ID NO: 2 and a second primer comprising a sequence of SEQ ID NO: 13, is used in the RNA amplification step, the oligonucleotide probe comprises a sequence consisting of, or being complementary to, at least 10 consecutive bases in SEQ ID NO: 34 is preferred.

In addition, other examples disclosed herein are a combination of a first primer of any one of SEQ ID NOs: 1 to 7, and a second primer of any one of SEQ ID NOs: 8 to 20 and an oligonucleotide probe comprising a sequence consisting of, or being complementary to at least 10 consecutive bases in SEQ ID NO: 34.

In this case, it is also preferred to chemically modify the les hydroxyl group at the 3' end of the probe (for example, by adding glycolic acid) to prevent an elongation reaction starting from this probe.

By carrying out the amplification method in the presence of the above-mentioned probe, amplification and detection of HIV-1 RNA can be carried out at constant temperature, in one tube, in one step and can be automated easily.

In the following, the present invention will be described in further detail by referring to Examples. However, the present invention is by no means restricted to these specific Examples. The examples illustrate the invention:

### EXAMPLE 1

The target HIV-RNA derived from various numbers of initial copies was detected using the combinations of oligonucleotides in the present invention.
(1) A 1628-nt RNA sequence of HIV-1 containing the structural gene of the core protein (gag) was used as a standard RNA. The standard RNA was obtained from HIV-1 RNA with ACCRUN^{™} 315 (product name), HIV-1 RNA Positive Control, Series 400 (BBI (Boston Biomedica, Inc.) by conventional extraction. Synthesis and subsequent purification of a double-stranded DNA containing the sequence of the gag region was performed by RT-PCR and in vitro transcription using the DNA as the template.
(2) The standard RNA was diluted with an RNA diluent (10 mM Tris-HCl (pH 8.0), 0.1 mM EDTA, 1 mM DTT, 0.5 U/µ 1 RNase inhibitor (Takara Shuzo Co., Ltd.)) to 10 copies/5 µl, 10⁴ copies/5 µl, 10³ copies/5 µl, 10² copies/5 µl and 10 copies/5 µl and quantified by UV absorption at 260mm. The diluent alone was used as a control sample (Nega).
(3) 20.8 µl portions of a reaction solution of the following composition were dispensed into 0.5 ml PCR tubes (Gene Amp Thin-Walled Reaction Tubes, Perkin Elmer), and 5 µℓ of the RNA sample at the above-mentioned concentrations was added.
The composition of the reaction solution (in terms of the concentrations in the final volume of 30 µl)

| | |
|---|---|
| 60 mM | Tris-HCl buffer (pH 8.6) |
| 13 mM | Magnesium chloride |
| 115 mM | Potassium chloride |
| 39 U | RNase Inhibitor |
| 1 mM | DTT |
| 0.25 mM | each of dATP, dCTP, dGTP and dTTP |
| 3.6 mM | ITP |
| 3.0 mM | each of ATP, CTP, GTP and UTP |
| 1.0 µM | First primer (SEQ ID NO:1) |
| 1.0 µM | Second primer (SEQ ID NO:15) (which additionally contained the nucleotide sequence of SEQ ID NO: 35 including the T7 promoter sequence at the 5' end (SEQ ID NO: 35 comprises the T7 promoter sequence from "A" at position 1 from the 5' end to "A" at position 22 and the subsequent enhancer sequence from "G" at position 23 to "A" at position 28) |
| 0.16 µM | Third oligonucleotide (SEQ ID NO:27) |
| 25 nM | Oligonucleotide (SEQ ID NO:34) labeled with a fluorescent intercalative dye (Fig. 1) (having the fluorescent intercalative dye between "T" at position 14 from the 5' end and "T" at position 15 and having a hydroxyl group modified with glycolic acid at the 3' end) |
| 13% | DMSO and |
| Distilled water for volume adjustment | |

(4) The reaction solutions were incubated at 41°C for 5 minutes, and 4.2 µl of an enzyme solution of the following composition, which was pre-incubated at 41°C for 2 minutes, was added. The composition of the enzyme solution (in terms of the concentration in the final volume of 30 µl) was:

| | |
|---|---|
| 1.7% | Sorbitol |
| 3 µg | Bovine serum albumin |
| 142 U | T7 RNA polymerase (GIBCO) |
| 8 U | AMV reverse transcriptase (Takara Shuzo Co.,Ltd.) and |
| Distilled water for volume adjustment | |

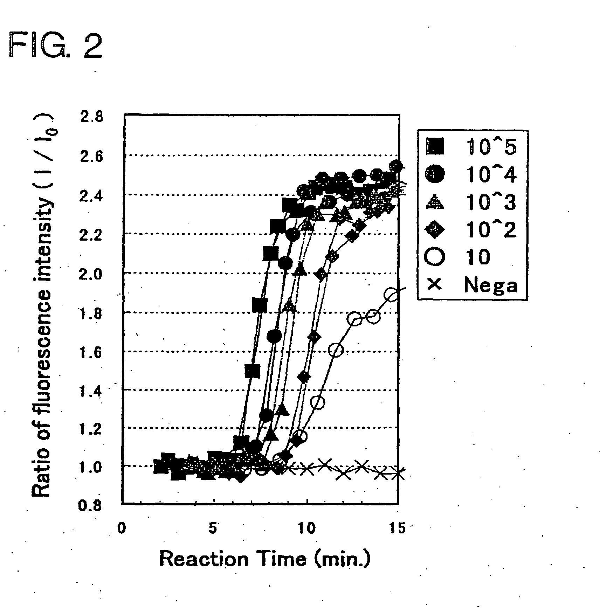

(5) The fluorescence intensities of the reaction solutions in the PCR tubes were directly monitored at 41°C in a thermostatic fluorescent spectrophotometer at an excitation wavelength of 470 nm and an emission wavelength of 510 nm. The time courses of the ratio of fluorescence intensities of the samples (fluorescence intensity at a certain time/background fluorescence intensity) from addition of the enzyme solution at 0 minute are shown in Fig. 2. The initial amounts of the RNA ranged from 10 copies/30 µl to 10⁵ copies/30 µl.

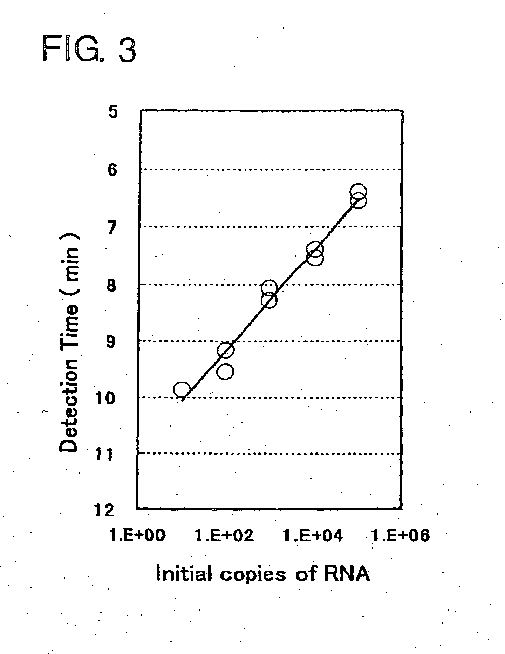

As shown in Fig. 2, the fluorescence profile was dependent on the initial concentration of the standard RNA, and it was possible to detect 10 copies in about 10 minutes. When the detection time, which is defined as the time at the ratio of fluorescent intensity of 1.2, was plotted as ordinate, and the initial RNA concentration was plotted as abscissa, a linear relation was found between them (Fig. 3). This result indicates that it is possible to quantify HIV-1 RNA in an unknown sample by using Fig. 3 as the calibration curve. Thus, it is proven that the present invention allows speedy and sensitive quantitative detection of HIV-1 RNA.

### EXAMPLE 2

HIV-1 RNA in the nucleic acid extracted from HIV-positive serum was detected using the combinations of oligonucleotides described in connection with the present invention.
(1) As the HIV-positive serum, ACCRUN 315 (product name), HIV-1 RNA Positive Control, Series 400 (BBI Boston Biomedica, Inc.) was used. HIV-1 RNA was obtained from 30 µl of the positive serum by conventional extraction and was diluted with an RNA diluent (10 mM Tris-HCl (pH 8.0), 0.1 mM EDTA, 1 mM DTT, 0.5 U/µ 1 RNase inhibitor(Takara Shuzo Co., Ltd.)) to an estimated RNA amount of 10³ copies/5 µl. The same standard RNA as in Example 1 was used at a concentration of 10³ copies/5 µl.
(2) A 20.8 8 µl portion of a reaction solution of the following composition was dispensed into a 0.5 ml PCR tube (Gene Amp Thin-Walled Reaction Tubes, Perkin Elmer), and 5 µℓ of the RNA sample at the above-mentioned concentration was added.
The composition of the reaction solution (in terms of the concentrations in the final volume of 30 µl)

| | |
|---|---|
| 60 mM | Tris-HCl buffer (pH 8.6) |
| 13 mM | Magnesium chloride |
| 115 mM | Potassium chloride |
| 39 U | RNase Inhibitor |
| 1 mM | DTT |
| 0.25 mM | each of dATP, dCTP, dGTP and dTTP |
| 3.6 mM | ITP |
| 3.0 mM | each of ATP, CTP, GTP and UTP |
| 1.0 µM | First primer (SEQ ID NO:2) |
| 1.0 µM | Second primer (SEQ ID NO:13) (which additionally contained the nucleotide sequence of SEQ ID NO: 35 including the T7 promoter sequence at the 5' end (SEQ ID NO:35 comprises the T7 promoter sequence from "A" at position 1 from the 5' end to "A" at position 22 and the subsequent enhancer sequence from "G" at position 23 to "A" at position 28)) |
| 0.16 µM | Third oligonucleotide (SEQ ID NO:26) |
| 25 nM | Oligonucleotide (SEQ ID NO:34) labeled with a fluorescent intercalative dye (Fig. 1) (having the fluorescent intercalative dye between "T" at position 14 from the 5' end and "T" at position 15 and having a hydroxyl group modified with glycolic acid at the 3' end) |
| 13% DMSO | and |
| Distilled water for volume adjustment | |

(3) The reaction solution was incubated at 41°C for 5 minutes, and 4.2 µℓ of an enzyme solution of the following composition which was pre-incubated at 41°C for 2 minutes, was added.
The composition of the enzyme solution (in terms of the concentrations in the final volume of 30 µl)

| | |
|---|---|
| 1.7% | Sorbitol |
| 3 µg | Bovine serum albumin |
| 142 U | T7 RNA polymerase (GIBCO) |
| 8 U | AMV reverse transcriptase (Takara Shuzo Co.,Ltd.) and |
| Distilled water for volume adjustment | |

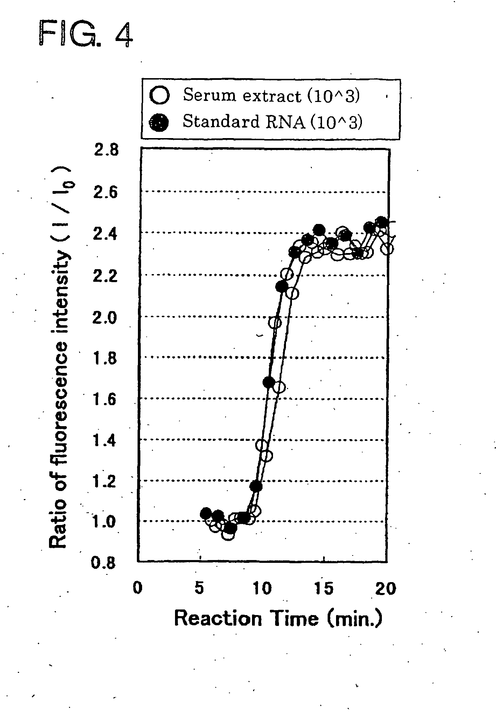

(4) The fluorescence intensity of the reaction solution in the PCR tube was directly monitored at 41°C in a thermostatic fluorescent spectrophotometer at an excitation wavelength of 470 nm and an emission wavelength of 510 nm. The time course of the ratio of fluorescence intensity of the sample (fluorescence intensity at a certain time/background fluorescence intensity) from the addition of the enzyme solution at 0 minute is shown in Fig. 4.

Fig. 4 demonstrates that it was possible to detect the RNA (at an estimated concentration of 10³ copies/30 µl) extracted from the HIV-positive serum at the same detection time as the standard RNA (RNA concentration: 10³ copies/30 µl) of the control.

Thus, it is proven that the present invention allows speedy and sensitive detection of HIV-1 RNA extracted from HIV-positive serum.

As described above, the present invention provides a simple, speedy and sensitive method of detecting HIV-RNA through provision of an oligonucleotide. This oligo-nucleotide can bind to a region of the genomic RNA of HIV-1 which is free of secondary structure. Thus, allowing binding at relatively low and constant temperatures (at 35°C to 50°C, preferably at 41°C) and use of the oligonucleotide primer for amplification of a.nucleic acid.

The entire disclosure of Japanese Patent Application No. 2001-129210 filed on April 26, 2001 including specification, claims, drawings and summary are incorporated herein by reference in its entirety.

## Claims

1. A method of amplifying an RNA of IDV -1 anticipated in a sample as a template, which comprises the steps of
(a) synthesizing a cDNA by the action of an RNA-dependent DNA polymerase by using a first primer containing a sequence complementary to a specific sequence of said RNA;
(b) denuding the cDNA of a single-stranded DNA through degradation of the RNA in the resulting RNA-DNA double strand by ribonuclease H activity;
(c) forming a double-stranded DNA, having a promoter sequence which can be transcribed into an RNA consisting of the specific sequence or a sequence complementary to the specific sequence, by using the single-stranded DNA as template, a second primer containing a sequence homologous to the specific sequence and by the action of a DNA-dependent DNA polymerase;
(d) transcribing a strand of the double-stranded DNA into an RNA transcript by the use of an RNA polymerase; and
(e) synthesizing a cDNA of the RNA transcript of step (d) by the RNA-dependent DNA polymerase, in the presence of the RNA polymerase,
wherein either the first or the second primer contains additionally a promoter sequence for the RNA polymerase at the 5' end, and wherein the first primer is an oligonucleotide comprising the sequence of SEQ ID NO: 2 and the second primer is an oligonucleotide comprising the sequence of SEQ ID NO: 13.

2. The method according to claim 1, which further comprises adding a third oligonucleotide which is complementary to a region of the RNA of HIV-1 which flanks the 5' end of the specific sequence with an overlap of from 1 to 10 bases with the specific sequence to form a template used in the initial stage of the amplification by cutting the RNA derived from HIV-1 at the 5' end of the specific sequence by the action of the ribonuclease H activity, wherein the first primer is an oligonucleotide of SEQ ID NO: 2, the second primer is an oligonucleotide of SEQ ID NO: 13, and the third oligonucleotide is an oligonucleotide of any one of SEQ ID NOS: 23 to 30.

3. A method of detecting HIV-1, which comprises conducting the method as defined in claim 1 or 2 in the presence of an oligonucleotide probe having a sequence different from those of the first primer and the second primer which can specifically bind to the RNA transcript resulting from the amplification and is labeled with a fluorescent intercalative dye, and measuring the change in the fluorsescence of the reaction solution, wherein said oligonucleotide probe (a) is designed to hybridize with at least part of the RNA transcript and to alter its fluorescence upon hybridization and (b) comprises a sequence consisting of, or being complementary to, at least 10 consecutive nucleotides in SEQ ID NO: 34.

4. Use of the oligonucleotides of SEQ ID NOS: 2 and 13 for the amplification of HIV-1 RNA.

5. Use of the oligonucleotides of SEQ ID NOS: 2, 13 and 34 for the detection of HIV-1 in a sample.

6. A kit comprising at least the oligonucleotides of SEQ ID NOS: 2, 13 and 34 in one or more containers.

## Patentansprüche

1. Verfahren zum Amplifizieren von RNA von HIV-1, das in einer Probe als Matrize vermutet wird, umfassend die Schritte:
(a) das Synthetisieren einer cDNA durch die Wirkung einer RNAabhängigen DNA-Polymerase unter Verwendung eines ersten Primers, der eine zu einer spezifischen Sequenz der RNA komplementäre Sequenz enthält;
(b) das Freilegen der cDNA einer einzelsträngigen DNA durch Abbau der RNA in dem entstehenden RNA-DNA-Doppelstrang durch Ribonuclease H-Aktivität;
(c) das Erzeugen einer doppelsträngigen DNA mit einer Promotorsequenz, die in eine RNA transkribiert werden kann, die aus der spezifischen Sequenz oder einer zu der spezifischen Sequenz komplementären Sequenz besteht, unter Verwendung der einzelsträngigen DNA als Matrize, eines zweiten Primers, der eine Sequenz enthält, die homolog zu der spezifischen Sequenz ist, und durch die Wirkung einer DNAabhängigen DNA-Polymerase;
(d) das Transkribieren eines Strangs der doppelsträngigen DNA in ein RNA-Transkript unter Verwendung einer RNA-Polymerase; und
(e) das Synthetisieren einer cDNA des RNA-Transkripts von Schritt (d) durch die RNA-abhängige DNA-Polymerase in Anwesenheit der RNA-Polymerase,
wobei entweder der erste oder der zweite Primer zusätzlich eine Promotorsequenz für die RNA-Polymerase am 5'-Ende enthält, und wobei der erste Primer ein Oligonucleotid ist, das die Sequenz von SEQ ID NO:2 umfasst, und der zweite Primer ein Oligonucleotid ist, das die Sequenz von SEO ID NO:13 umfasst.

2. Verfahren nach Anspruch 1, zusätzlich umfassend das Zugeben eines dritten Oligonucleotids, das komplementär zu einer Region der RNA von HIV-1 ist, die das 5'-Ende der spezifischen Sequenz mit einer Überlappung von einer bis 10 Base(n) mit der spezifischen Sequenz flankiert, um durch Schneiden der von HIV-1 stammenden RNA am 5'-Ende der spezifischen Sequenz durch die Wirkung der Ribonuclease H-Aktivität eine Matrize zu erzeugen, die in der Anfangsphase der Amplifikation verwendet wird, wobei der erste Primer ein Oligonucleotid von SEQ ID NO:2 ist, der zweite Primer ein Oligonucleotid von SEQ ID NO:13 ist und das dritte Oligonucleotid ein Oligonucleotid von einer der SEQ ID NO:23 bis 30 ist.

3. Verfahren zum Nachweis von HIV-1, umfassend das Durchführen des Verfahrens wie in Anspruch 1 oder 2 definiert, in Anwesenheit einer Oligonucleotidsonde mit einer Sequenz, die unterschiedlich ist zu der des ersten Primers und des zweiten Primers, und die spezifisch an das RNA-Transkript binden kann, das aus der Amplifikation entsteht und die mit einem fluoreszierenden, interkalierenden Farbstoff markiert ist, und das Messen der Änderung in der Fluoreszenz der Reaktionslösung, wobei die Oligonucleotidsonde (a) konzipiert ist, um mit zumindest einem Teil des RNA-Transkripts zu hybridisieren und seine Fluoreszenz nach Hybridisierung zu ändern, und (b) eine Sequenz umfasst, bestehend aus, oder komplementär zu, zumindest 10 aufeinanderfolgende Nucleotiden in SEQ ID NO:34.

4. Verwendung der Oligonucleotide von SEQ ID NO:2 und SEQ ID NO:13 für die Amplifizierung von HIV-1-RNA.

5. Verwendung der Oligonucleotide von SEQ ID NO:2, 13 und 34 für den Nachweis von HIV-1 in einer Probe.

6. Kit, der zumindest die Oligonucleotide von SEQ ID NO:2, 13 und 34 in einem oder mehreren Behälter(n) umfasst.

## Revendications

1. Procédé d'amplification d'un ARN de VIH-1 prévu dans un échantillon en tant que matrice, qui comprend les étapes consistant à :
(a) synthétiser un ADNc par l'action d'une ADN polymérase ARN dépendante en utilisant une première amorce contenant une séquence complémentaire à une séquence spécifique dudit ARN ;
(b) dénuder l'ADNc d'un ADN simple brin par dégradation de l'ARN dans le double brin ARN-ADN résultant par une activité de ribonucléase H ;
(c) former un ADN double brin, ayant une séquence promoteur qui peut être transcrite en un ARN constituée de la séquence spécifique ou d'une séquence complémentaire à la séquence spécifique, en utilisant l'ADN simple brin en tant que matrice, une seconde amorce contenant une séquence homologue à la séquence spécifique et par l'action d'une ADN polymérase ADN dépendante ;
(d) transcrire un brin de l'ADN double brin en un produit de transcription ARN par l'utilisation d'une ARN polymérase ; et
(e) synthétiser un ADNc du produit de transcription ARN de l'étape (d) par l'ADN polymérase ARN- dépendante, en présence de l'ARN polymérase,
dans lequel soit la première amorce soit la seconde amorce contient de plus une séquence promoteur pour l'ARN polymérase à l'extrémité 5', et dans lequel la première amorce est un oligonucléotide comprenant la séquence SEQ ID NO: 2 et la seconde amorce est un oligonucléotide comprenant la séquence SEQ ID NO: 13.

2. Procédé selon la revendication 1, qui comprend en outre l'ajout d'un troisième oligonucléotide qui est complémentaire à une région de l'ARN de VIH-1 qui flanque l'extrémité 5' de la séquence spécifique avec un chevauchement de 1 à 10 bases avec la séquence spécifique pour former une matrice utilisée au stade initial de l'amplification en coupant l'ARN issu du VIH-1 à l'extrémité 5' de la séquence spécifique par l'action de l'activité de ribonucléase H, dans lequel la première amorce est un oligonucléotide de SEQ ID NO: 2, la seconde amorce est un oligonucléotide de SEQ ID NO: 13 et le troisième oligonucléotide est un oligonucléotide ayant l'une quelconque des séquences SEQ ID NOS: 23 à 30.

3. Procédé de détection de VIH-1, qui comprend la mise en oeuvre du procédé tel que défini dans la revendication 1 ou 2 en présence d'une sonde oligonucléotidique ayant une séquence différente de celles de la première amorce et de la seconde amorce qui peut se lier spécifiquement au produit de transcription ARN résultant de l'amplification et qui est étiquetée avec un colorant d'intercalation fluorescent, et la mesure du changement de fluorescence de la solution réactionnelle, dans lequel ladite sonde oligonucléotidique (a) est conçue de manière à s'hybrider à au moins une partie du produit de transcription ARN et à altérer sa fluorescence lors de l'hybridation et (b) comprend une séquence constituée de, ou complémentaire à, au moins 10 nucléotides consécutifs dans SEQ ID NO: 34.

4. Utilisation des oligonucléotides de SEQ ID NOS: 2 et 13 pour l'amplification de l'ARN de VIH-1.

5. Utilisation des oligonucléotides de SEQ ID NOS: 2, 13 et 34 pour la détection de VIH-1 dans un échantillon.

6. Kit comprenant au moins les oligonucléotides de SEQ ID NOS: 2, 13 et 34 dans un ou plusieurs récipients.
